# EUROPEAN PATENT SPECIFICATION

(11) **EP 1 478 876 B1**
(45) Date of publication and mention of the grant of the patent: **21.09.2005**
(21) Application number: 03711299.2
(22) Date of filing: 25.02.2003
(51) Int. Cl.: F21S 8/00, F21V 21/03, F16M 11/06, E04B 9/00

(54) **SURGICAL SUSPENSION SYSTEM**
CHIRURGISCHE AUFHÄNGEVORRICHTUNG
SYSTEME DE SUSPENSION CHIRURGICAL

(30) Priority: 25.02.2002 US 359518 P
(43) Date of publication of application: 24.11.2004
(73) Proprietor: STERIS INC., California 92590 (US); ONDAL INDUSTRIETECHNIK GMBH, 36085 Hunfeld (DE)
(72) Inventor: BRAHLER, Manfred, 36137 Grossenlüder (DE); STEGER, Klaus, 36132 Eiterfled (DE); HLEBOVY, James, C., Chardon, OH 44024 (US)
(74) Representative: Gasquet, Denis
(86) International application number: PCT/US2003/006110
(87) International publication number: WO 2003/072996

(56) References cited:
- EP-A- 1 067 419
- DE-A- 3 312 137
- DE-A- 19 739 733
- FR-A- 2 678 030
- US-A- 5 090 654

## Description

### Background of the Invention

The present invention relates to the suspension system arts. It particularly relates to suspension systems for surgical operating room lightheads, monitors, cameras, and the like, and will be described with particular reference thereto. However, the invention will also find application in other ceiling-mounted apparatus and in fields outside the medical industry.

In operating theaters, intensive care rooms, and other hospital and clinical settings, medical equipment, such as overhead lighting and monitoring devices, is carried from overhead by suspension systems extending downward from a ceiling. This arrangement advantageously places the equipment out of the way of busy medical personnel and yet readily accessible when needed. Suspended lighting, for example, can effectively illuminate the surgical site without physically interfering with the surgeon.

Such suspension systems usually include a mounting plate (sometimes called a "cheese plate") attached to a rigid overhead structure, a drop tube connected to the mounting plate, a rotatable spindle fixed to the drop tube which allows rotation about a vertical axis, and one or a plurality of extension and/or articulating arms which connect with and support equipment such as surgical lightheads, monitors, cameras, or other devices. The articulating arms are often multiply jointed to permit several degrees of mechanical freedom for the attached device.

The connection of the drop tube to the mounting plate most often uses a tube-in-tube design wherein the drop tube is fixed to a cylinder which is in turn fixed to the mounting plate using screws or other suitable fasteners. Because ceiling heights in hospitals and clinics vary from one facility to another, and because the suspension system preferably accommodates placement of medical devices in ergonomically acceptable positions for medical personnel relative to the floor, the suspension systems are advantageously adaptable for different ceiling heights.

However, existing suspension systems typically use a drop tube having a fixed length. Height adjustment of the overall system is accomplished either by selecting a drop tube of an appropriate standard or custom length, or by cutting the tube at the installation site and drilling the necessary holes into the tube at the proper locations to effect secure attachment.

Providing pre-selected custom length drop tubes that are pre-cut at the factory to match the ceiling height disadvantageously introduces logistical problems, long lead times, and the possibility that the drop tubes will not fit with the actual relative ceiling to floor spacing.

Cutting a tube at the installation site risks poorly executed cutting and/or drilling of the tube resulting in a damaged suspension system and possible safety issues. Another disadvantage of cutting the tube at the installation site is that it is usually not possible to machine properly the end of the tube which is cut. The rough cut end is accommodated by including relatively large tolerances for the tube-in-tube connection and may require adjustment screws or the like. However, abnormal clearances can nonetheless result and cannot always be corrected by the adjustment screws.

Yet another disadvantage of existing suspension systems is that the height of the finished system is not subsequently adjustable in the vertical direction. Thus, when the suspension system is moved to a different operating theater having a different ceiling height the drop tube is either replaced or, if the new operating theater has a lower ceiling, re-cut to accommodate the lower ceiling.

The present invention contemplates an improved surgical suspension apparatus which overcomes the aforementioned limitations and others.

### Summary of the Invention

According to one aspect of the invention, a suspension system for suspending one or more of lightheads, monitors, task lights, cameras, and other medical apparatus from an overhead structure at a selectable height is provided. The suspension system includes a drop tube configured for supporting the one or more of lightheads, monitors, cameras, and other medical apparatus. A receiving element receives the drop tube such that a distal end of the drop tube extends below a lower end of the receiving element. A collar is received by the receiving element and surrounds a portion of the drop tube to effectuate a compressive clamping of the portion of the drop tube inside the receiving element, a distance of the drop tube which extends below the lower end of the receiving element being variable. A means is provided for mounting the receiving element to the overhead structure.

According to another aspect of the invention, a method of variably adjusting a distance, relative to a fixed surface, of a distal end of a drop tube for supporting a medical device is provided. The method includes rigidly supporting a receiving element from the fixed surface. The method further includes loosely inserting a collar into a bore of the receiving element, inserting the drop tube into the collar such that an end of the drop tube extends a selected distance below the receiving element, the distance of the drop tube which extends below a lower end of the receiving element being variable, and drawing the collar into the receiving element bore such that the drop tube is compressively clamped by the receiving element and collar.

One advantage of the present invention resides in the enablement of continuous height adjustment over a range of positions in installing a surgical suspension system.

Another advantage of the present invention is the ability to adapt the suspension system height to different surgical theaters or other ceiling height changes.

Another advantage of the present invention resides in the elimination of circumferential tube-in-tube fitting clearances which are replaced in the preferred embodiment of the invention by a compressed wedge element that surroundingly clamps onto the drop tube.

Yet another advantage of the present invention is the elimination of on-site installation work including precision tube cutting and drilling. The drop tube can be "rough cut" at the installation site to provide a desired nominal tube length, but precision machining is not necessary.

Still yet another advantage of the present invention is the elimination of a precise length specification in pre-selected custom length drop tubes.

Numerous additional advantages and benefits of the present invention will become apparent to those of ordinary skill in the art upon reading the following detailed description of the preferred embodiment.

### Brief Description of the Drawings

The invention may take form in various components and arrangements of components, and in various steps and arrangements of steps. The drawings are only for the purpose of illustrating preferred embodiments and are not to be construed as limiting the invention.
FIGURE 1 shows a plurality of medical devices including lightheads and monitoring equipment connected to a suspension system formed in accordance with an embodiment of the invention;
FIGURE 2 shows an exploded sectional view of a suspension system formed in accordance with an embodiment of the invention;
FIGURE 3 shows an assembled sectional view of the suspension system of FIGURE 2;
FIGURE 4 shows an exploded perspective view of the suspension system of FIGURES 2 and 3 with additional ornamental cover plate and connecting components; and
FIGURE 5 shows an assembled view, in partial section, of the suspension system of FIGURE 3 with a safety ring assembly attached to the drop tube.

### Detailed Description of the Preferred Embodiments

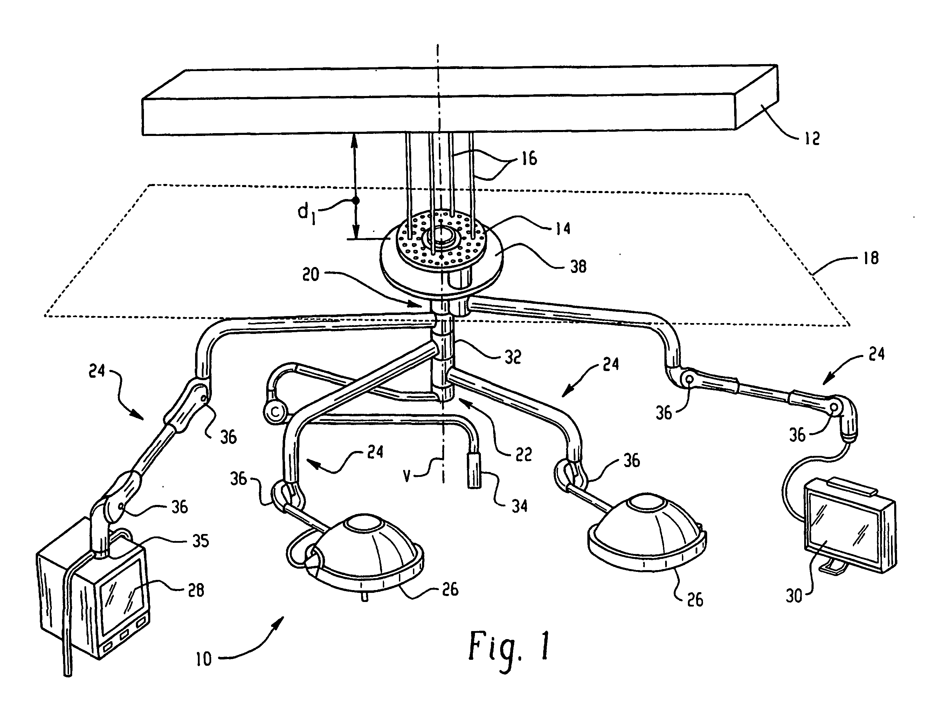

With reference to FIGURE 1**,** an overhead lighting system **10** is mounted from a fixed overhead structure **12** which in the illustrated case is an overhead beam **12.** A mounting plate or "cheeseplate" **14** is secured at a selected distance **d**_{**1**} below the overhead structure **12** by a plurality of long-shank fasteners **16.** Typically, the distance **d**₁ corresponds to a distance of an operating room ceiling **18** below the structural component **12.**

A drop tube **20** connects at a proximal end to the mounting plate **14** and has a distal end **22** extending downward. One or more articulating arms **24** are disposed at the distal end **22.** Each articulating arm **24** has a medical device, such as a lighthead **26,** CRT monitor **28,** flat panel monitor **30,** manual task light **34,** or the like attached at a distal end **35.** Typically, the proximal end of each articulating arm **24** connects to the drop tube **20** via a rotatable spindle **32** which is rotatable about a vertical axis **V**. Each articulating arm **24** usually includes one or multiple joints **36** which are adjustable about one or more axes each to provide additional degrees of motion freedom.

The overhead lighting system **10** optionally includes additional features, such as a cosmetic ceiling cover **38.** Those skilled in the art will also appreciate that the system **10** can be employed for mounting other devices besides lightheads and monitors, such as cameras, fiber optical light pipes, and the like. It will further be appreciated that the overhead lighting system **10** is not limited in application to surgical theaters, or even to medical or clinical settings. The overhead mounting of devices is beneficial in any setting where it is advantageous to have devices conveniently accessible and yet not "in the way" of people's usual movements.

An important parameter of the overhead lighting system **10** is the height of the distal end **22** of the drop tube **20** relative to the floor. For example, many surgical lightheads include reflectors designed to reflect light around the surgeon's head. The lighthead is thus positioned behind the surgeon's head, and the light reflects around the surgeon's head and onto or into the surgical opening. The precise positioning of the lighthead relative to the surgeon's head is thus critical, and improper positioning can result in partial blockage of the illumination by the surgeon's head, or a collision. Similarly, the monitors **28, 30** should be placed at an ergonomically advantageous position so that the surgeon can easily view the monitors during the operation, with the ability to glance back-and-forth between the surgical opening and the monitors.

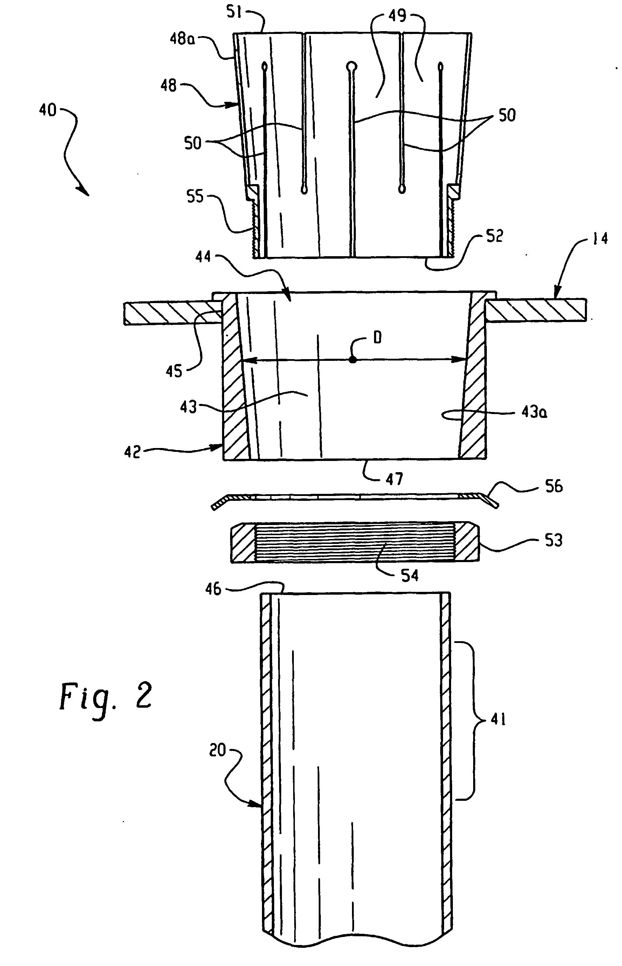

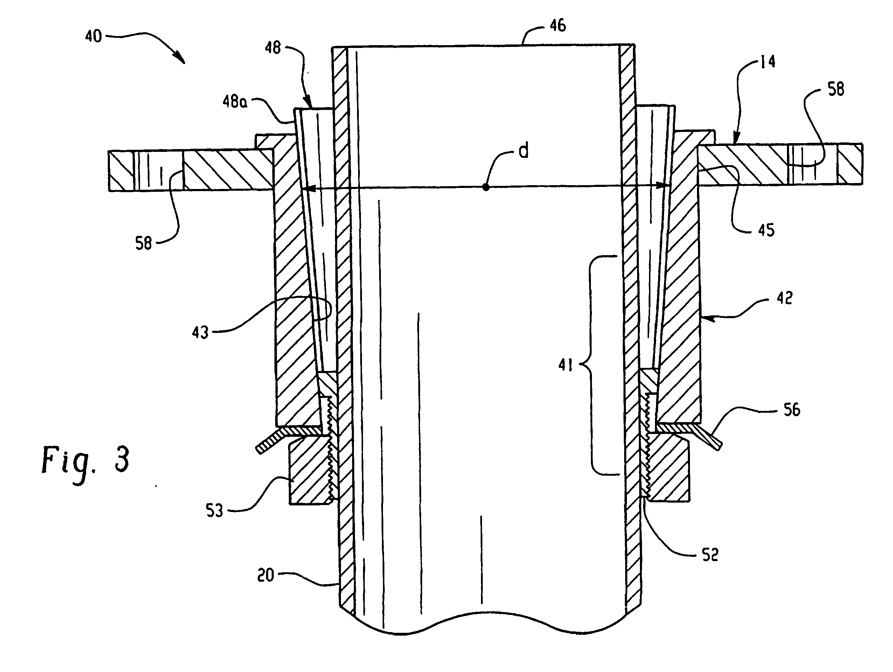

With reference to FIGURES 2 and 3, an adjustably positionable drop tube locking mechanism **40** is described. The locking mechanism **40** selectively clamps an intermediate portion **41** of the drop tube between the distal and proximal ends. The locking mechanism includes a drop tube receiving element in the form of a cylinder **42** with an axially extending bore **43,** with an interior wall surface **43a,** which defines an upper opening **44.** The bore **43** preferably has an inner diameter **D** which decreases, towards a lower end. The cylinder is thus internally shaped as a frustum of a cone and is rigidly attached inside a centrally located opening **45** of the mounting plate **14,** for example by welding. The mounting plate **14** together with the welded cylinder **42** provide a securing element **14, 42** for rigidly securing the drop tube **20** (shown in part in FIGURES 2 and 3). As best seen in the assembled view of FIGURE 3, the drop tube **20** passes through the opening **44** and narrowing bore **43.** The drop tube **20** has a distal end **22** (FIGURE 1) extending downward below the cylinder **42,** and a proximal end **46** extending upward adjacent the cylinder **42.** The drop tube **20** has a smaller outer diameter than the narrowest portion of the narrowing bore **43** and is therefore adjustably positionable within the cylinder **42** with its distal end **22** extending a selected distance below a lower narrow open end **47** of the bore.

Although a separate drop tube receiving element, namely the cylinder **42,** is shown in the illustrated embodiment, it is also contemplated to form the mounting plate **14** and the cylinder **42** as a single integral piece. That is, in a contemplated alternative embodiment the mounting plate includes an opening corresponding to the opening **44.** However, as can be discerned from FIGURES 2 and 3, such an alternative embodiment may include a thicker mounting plate.

With continuing reference to FIGURES 2 and 3, a locking tube or collar **48** surroundingly encompasses the drop tube **20.** The collar **48** is preferably wedge-shaped and has an outside surface **48a** with a diameter **D** which decreases toward a lower end thereof. The collar is thus essentially shaped as a frustum of a cone and is similarly sized to the similarly shaped narrowing bore **43** of the cylinder **42.** The taper of the outer surface may be the same as the taper of the bore, although it is also contemplated that the taper may be somewhat greater or lesser than that of the bore. The collar **48** includes at least one and preferably a plurality of longitudinally extending slots **50** spaced apart along the outer surface of the collar **48.** As shown in FIGURE 2, some of the slots **50** extend downward from a wider or upper end **51** of the collar, while other slots extend upward from a narrow or lower end **52** of the collar, although it is also contemplated that the slots need not extend fully to either end **51, 52.** The resiliently flexible strips **49** thus defined between the slots are able to flex inwardly when the **48** collar is exteriorly compressed. Where the strips **49** extend fully to one or other end **51, 52,** this allows the free ends of the strips to move into a somewhat overlapping relation when compressed.

The wedge-shaped collar **48** acts as a collet which passes through the cylinder opening **44** and is wedged into the bore **43,** with the lower end **52,** extending slightly beyond the lower open end **47** of the bore **43.** The wedging compresses the collar **48,** with the slots **50** facilitating the compression. During compression, the inner diameter of the collar decreases. As the collar **48** compresses, it presses against the drop tube **20** to effectuate a compressive clamping of the drop tube **20** inside the cylinder **42.** The collar **48** is preferably formed from metal of a sufficient thickness for the strips **49** to flex inward when compressed and return to their original positions when released.

To enable a secure compressive locking, a tightening nut **53** preferably is used. The nut **53** is internally threaded at **54** and threadedly attaches to external threads **55** disposed on the narrow end **52** of the collar **48.** As can be seen from FIGURE 2, the narrow end **52** is not tapered, in the region of the threads. As the nut **53** is tightened, the collar **48** is compressively drawn into the opening **44** of the cylinder **42** to effectuate the compressive clamping. In place of threads, other means of tightening the nut onto the collar are also contemplated. Optionally, a lock washer **56** is included to prevent the nut **53** from loosening. Furthermore, although a slotted wedge-shaped collar **48** is illustrated, other wedge-shaped elements are suitably substituted therefore as desired. For example, a collet or other type of locking tube is also contemplated. In another embodiment, only one of the collar **42** and cylinder **42** has a taper, for example, the cylinder bore **43** may have a constant diameter **D** while the collar is tapered, or the collar have a constant diameter d while the cylinder **42** is tapered.

FIGURE 3 particularly illustrates the subject wedge lock mechanism **40** and selected suspension system components in their assembled configuration. As can be appreciated, by tightening the nut **53,** the wedge **48** is drawn further into the narrowing bore **43** of the securing element **14, 42** and is clamped into place. The slots **50** within the wedge **48** allow the wedge **48** to compress and tighten around the outer surface of the drop tube **20.** The drop tube **20** is essentially thereby fixed within the wedge-shaped collar **48** and the cylinder **42** through frictional forces created between the component parts. The wedge lock mechanism **40** advantageously provides for continuous and repeatable height adjustment, by simply loosening the nut **53,** sliding the drop tube **20** to its new position relative to the cylinder **42** and, more importantly, to the floor, and retightening the nut **53.**

The length of drop tube **20** which extends below the cylinder **42** is thus infinitely variable between an upper position, in which the uppermost spindle **32** is flush with the nut **53,** and a lower position, in which the upper end of the tube **20** is clamped by the collar. The excess, upper portion of the drop tube **20** is thus "stored" until needed within the cylinder **42** and may extend upward, into the space above the ceiling **18,** thus providing for the portion of the drop tube below the cylinder **42** to be increased or decreased in length, as the need arises.

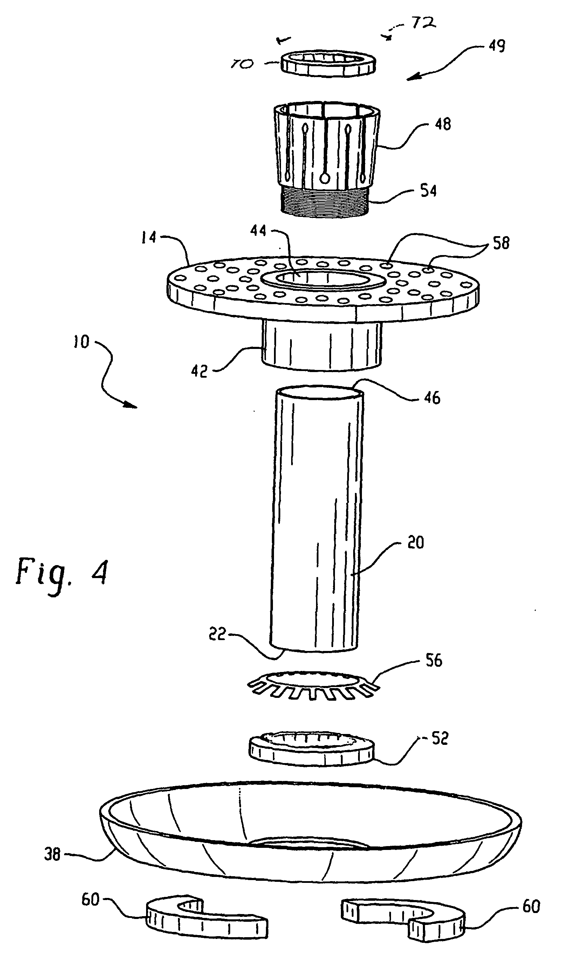

With continuing reference to FIGURES 1 through 3 and with further reference to FIGURE 4 which shows a perspective exploded view of the suspension system **10,** the suspension system **10** is connected using the fasteners **16** (FIGURE 1) which insert into a selected plurality of the openings **58** in the cheeseplate **14.** The cosmetic ceiling cover **38** (FIGURES 1 and 4) is secured by locking half-rings 60 (FIGURE 4). Since the cosmetic ceiling cover **38** is not a weight-bearing component, the securing does not need to be particularly strong, and various securing components are contemplated in place of the rings **60.**

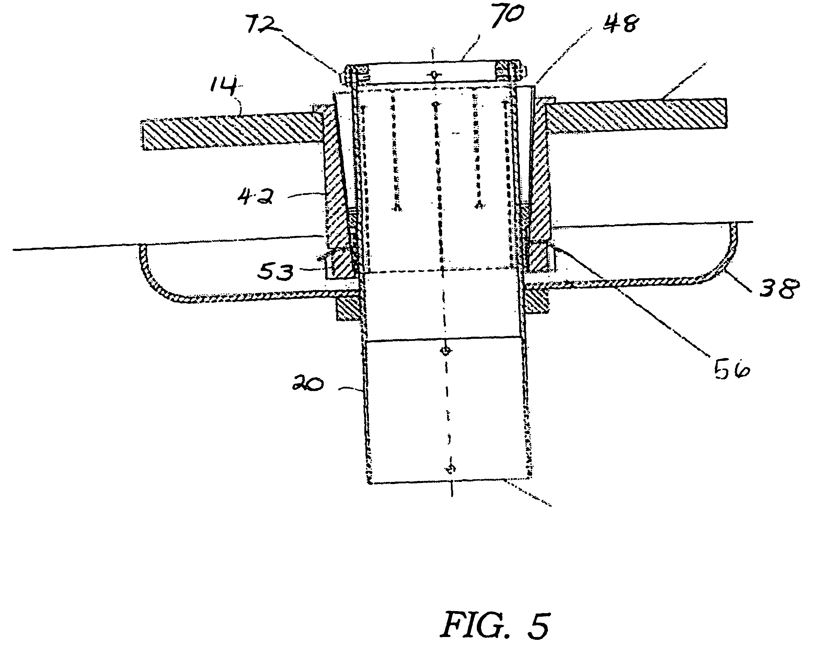

Preferably, a safety ring assembly **70** is provided to prevent the tube **20** from pulling through the collar **48** in the unlikely event that the system loosens. The safety ring assembly attaches to the tube **20** above the collar **48** using suitable fasteners **72,** such as bolts, screws or the like (FIGURE 5). In that way, if the tube **20** slides relative to the collar **48**, contact is made between the split rings of the safety ring and the upper end of the tapered collar urging the collar into further compression against the tube and also mechanically preventing the tube from pulling through the collar.

## Claims

1. A suspension system **(10)** for suspending one or more of lightheads **(26),** monitors **(28, 30),** task lights **(34)**, cameras, and other medical apparatus from an overhead structure **(12)** at a selectable height, the suspension system comprising a drop tube **(20)** configured for supporting the one or more of lightheads, monitors, cameras, and other medical apparatus, **characterized by**:
a receiving element **(42)** which receives the drop tube such that a distal end **(22)** of the drop tube extends below a lower end **(47)** of the receiving element;
a collar **(48)** which is received by the receiving element and surrounds a portion **(41)** of the drop tube to effectuate a compressive clamping of the portion of the drop tube that is inside the receiving element such that a length of the drop tube which extends below the lower end of the receiving element is variable; and
means **(14,16)** for mounting the receiving element to the overhead structure.

2. The suspension system of claim 1, further **characterized by**:
the means for mounting including a mounting plate **(14)** rigidly connected with the receiving element and configured for mounting to the overhead structure, the mounting plate having an opening **(45)** through which an end of the drop tube passes.

3. The suspension system of claim 1 or claim 2, further **characterized by**:
the receiving element having an inside diameter **D** which decreases toward the lower end.

4. The suspension system of claim 3, further **characterized by**:
the receiving element inside diameter being defined by a generally frustoconical bore **(43).**

5. The suspension system of any one of preceding claims 1-4, further **characterized by**:
the collar having an outer diameter d which decreases toward a lower end **(52)** thereof.

6. The suspension system of claim 5, further **characterized by**:
the outer diameter of the collar being greater than a minimum inside diameter of the receiving element.

7. The suspension system of any one of preceding claims 1-6, further **characterized by**:
a tightening nut **(53)** adapted to thread onto a portion **(55)** of the -collar to compressively draw the collar into the receiving element.

8. The suspension system of any one of preceding claims 1-7, further **characterized by**:
the collar including at least one longitudinally extending slot **(50).**

9. The suspension system of claim 8, further **characterized by**:
the collar including a plurality of longitudinally extending slots.

10. The suspension system of either one of preceding claims 8 and 9, further **characterized by**:
the at least one slot defining a flexible strip **(49)** which deflects radially inward when compressive exterior force is applied to the collar.

11. The suspension system of any one of preceding claims 1-10, further **characterized by**:
the drop tube comprising a cylinder of generally uniform exterior diameter.

12. The suspension system of any one of preceding claims 1-11, further **characterized by**:
a rotatable spindle **(32)** disposed at a lower end of the drop tube.

13. The suspension system of claim 12, further **characterized by**:
an articulating arm **(24)** attached to the rotatable spindle, the articulating arm having at least one adjustable joint **(36)** and an attachment end **(35)** adapted to receive one of the lightheads, monitors, task lights, cameras, and other medical apparatus.

14. The suspension system of any one of preceding claims 1-13, further **characterized by**:
a length of the distal end of the drop tube which extends below the receiving element being infinitely variable between upper and lower limits.

15. The suspension system of any one of preceding claims 1-14, further **characterized by**:
the collar having a substantially frustoconical exterior surface **(48a),** the frustoconical exterior surface engaging a substantially frustoconical interior surface **(43a)** of the receiving element.

16. The suspension system of any one of preceding claims 1-14, further **characterized by**:
the collar having a bore shaped to receive the drop tube slidingly therethrough, absent a compressive force being applied to the collar.

17. The suspension system of any one of preceding claims 1-16, further **characterized by**:
the collar including:
an outer surface of narrowing diameter terminating at a narrow end, which outer surface compressively wedges at least partially into an axial bore of the receiving element; and
an inner surface which compresses against the drop tube at a selected point responsive to the wedging to secure the drop tube in the drop tube bore at the selected point.

18. A method of variably adjusting a distance, relative to a fixed surface, of a distal end of a drop tube for supporting a medical device, the method comprising rigidly supporting a receiving element **(42)** from the fixed surface, the method **characterized by**:
loosely inserting a collar **(48)** into a bore **(43)** of the receiving element;
inserting the drop tube into the collar such that an end **(22)** of the drop tube extends a selected distance below the receiving element, the distance of the drop tube which extends below a lower end **(47)** of the receiving element being variable; and
drawing the collar into the receiving element bore such that the drop tube is compressively clamped by the receiving element and collar.

19. The method of claim 18, further **characterized by**:
the step of drawing the collar into the bore including tightening a nut **(53)** on a portion **(55)** of the collar.

20. The method of either one of claims 18 and 19, further **characterized by**:
the collar including a plurality of longitudinally extending, spaced slots **(50),** step of drawing the collar into the bore including compressing portions **(49)** of the collar defined between the slots.

## Patentansprüche

1. Aufhängungssystem **(10)** zum Aufhängen einer oder mehrerer Leuchten **(26),** Monitore **(28, 30),** Arbeitsleuchten **(34),** Kameras und weiterer medizinischer Geräte in einer wählbaren Höhe an eine oben gelegene Struktur **(12),** wobei das Aufhängungssystem ein vertikales Rohr **(20)** zum Tragen einer oder mehrerer der Leuchten, Monitore, Kameras und weiterer medizinischer Geräte aufweist, **gekennzeichnet durch** :
ein Aufnahmeelement **(42),** das das vertikale Rohr derart aufnimmt, dass das distale Ende **(22)** des vertikalen Rohrs sich unterhalb eines unteren Endes **(47)** des Aufnahmeelements erstreckt;
eine Manschette **(48),** die **durch** das Aufnahmeelement aufgenommen wird und einen Abschnitt **(41)** des vertikalen Rohrs derart umgibt, dass sie den sich innerhalb des Aufnahmeelements befindlichen Abschnitt des vertikalen Rohrs zusammenpressend einklemmt, so dass eine Länge des vertikalen Rohrs, die sich unterhalb des unteren Endes des Aufnahmeelements erstreckt, variierbar ist; und
Mittel **(14, 16)** zum Montieren des Aufnahmeelements an die oben gelegene Struktur.

2. Aufhängungssystem nach Anspruch 1, weiterhin **dadurch gekennzeichnet, dass**
die Mittel zum Montieren eine Montageplatte **(14)** umfassen, die mit dem Aufnahmeelement steif verbunden und an der oben gelegenen Struktur montierbar ist, wobei die Montageplatte eine Öffnung **(45)** hat, durch welche ein Ende des vertikalen Rohrs durchgeführt ist.

3. Aufhängungssystem nach Anspruch 1 oder 2, weiterhin **dadurch gekennzeichnet, dass**
das Aufnahmeelement einen in Richtung unteres Ende abnehmenden Innendurchmesser D hat.

4. Aufhängungssystem nach Anspruch 3, weiterhin **dadurch gekennzeichnet, dass**
der Innendurchmesser des Aufnahmeelements durch eine insgesamt kegelstumpfförmige Bohrung **(43)** definiert ist.

5. Aufhängungssystem nach einem der vorangegangenen Ansprüche 1-4, weiterhin **dadurch gekennzeichnet, dass**
die Manschette einen in Richtung ihres unteren Endes **(52)** abnehmenden Außendurchmesser d hat.

6. Aufhängungssystem nach Anspruch 5, weiterhin **dadurch gekennzeichnet, dass**
der Außendurchmesser der Manschette größer ist als der kleinste Innendurchmesser des Aufnahmeelements.

7. Aufhängungssystem nach einem der vorangegangenen Ansprüche 1-6, weiterhin **gekennzeichnet durch**
eine Anziehmutter **(53),** die auf einen Abschnitt **(55)** der Manschette aufgeschraubt werden kann, um die Manschette in das Aufnahmeelement zusammendrückend hineinziehen.

8. Aufhängungssystem nach einem der vorangegangenen Ansprüche 1-7, weiterhin **dadurch gekennzeichnet, dass**
die Manschette mindestens einen sich in Längsrichtung erstreckenden Schlitz **(50)** umfasst.

9. Aufhängungssystem nach Anspruch 8, weiterhin **dadurch gekennzeichnet, dass**
die Manschette mehrere sich in Längsrichtung erstreckende Schlitze umfasst.

10. Aufhängungssystem nach einem der vorangegangenen Ansprüche 8 und 9, weiterhin **dadurch gekennzeichnet, dass**
der mindestens eine Schlitz einen flexiblen Streifen **(49)** definiert, der bei einem auf die Manschette ausgeübten äußeren Druck radial nach innen ausgelenkt wird.

11. Aufhängungssystem nach einem der vorangegangenen Ansprüche 1-10, weiterhin **dadurch gekennzeichnet, dass**
das vertikale Rohr einen Zylinder mit einem insgesamt gleichmäßigen Außendurchmesser aufweist.

12. Aufhängungssystem nach einem der vorangegangenen Ansprüche 1-11, weiterhin **gekennzeichnet durch**:
eine an einem unteren Ende des vertikalen Rohrs angeordnete drehbare Spindel **(32).**

13. Aufhängungssystem nach Anspruch 12, weiterhin **dadurch gekennzeichnet, dass**
ein Gelenkarm **(24)** an der drehbaren Spindel angebracht ist, wobei der Gelenkarm mindestens ein verstellbares Gelenk **(36)** und ein Befestigungsende **(35)** zur Aufnahme eines der Leuchten, der Monitore, der Arbeitsleuchten, der Kameras und der weiteren medizinischen Geräte hat.

14. Aufhängungssystem nach einem der vorangegangenen Ansprüche 1-13, weiterhin **dadurch gekennzeichnet, dass**
eine Länge des distalen Endes des vertikalen Rohrs, das sich unterhalb des Aufnahmeelements erstreckt, zwischen einer oberen und einer unteren Grenze unendlich variierbar ist.

15. Aufhängungssystem nach einem der vorangegangenen Ansprüche 1-14, weiterhin **dadurch gekennzeichnet, dass**
die Manschette eine im Wesentlichen kegelstumpfförmige Außenfläche **(48a)** hat, wobei die kegelstumpfförmige Außenfläche mit einer im Wesentlichen kegelstumpfförmigen Innenfläche **(43a)** des Aufnahmeelements in Eingriff steht.

16. Aufhängungssystem nach einem der vorangegangenen Ansprüche 1-14, weiterhin **dadurch gekennzeichnet, dass**
die Manschette eine Bohrung hat, die derart geformt ist, dass das vertikale Rohr darin verschieblich aufgenommen werden und durch diese hindurchgreifen kann, ohne dass auf die Manschette eine Druckkraft ausgeübt wird.

17. Aufhängungssystem nach einem der vorangegangenen Ansprüche 1-16, weiterhin **dadurch gekennzeichnet, dass**
die Manschette umfasst:
eine Außenfläche mit einem abnehmenden Durchmesser, die mit einem schmalen Ende abschließt, wobei die Außenfläche zumindest teilweise in einer axialen Bohrung des Aufnahmeelements zusammenpressend eingeklemmt ist; und
eine Innenfläche, die infolge des Einklemmens an einem ausgewählten Punkt gegen das vertikale Rohr drückt, so dass das vertikale Rohr am ausgewählten Punkt in der Bohrung für das vertikale Rohr gesichert ist.

18. Verfahren zum variablen Verstellen eines Abstands eines distalen Endes eines vertikalen Rohrs zu einer festen Fläche zum Tragen eines medizinischen Geräts, wobei das Verfahren den Schritt umfasst, der darin besteht, ein Aufnahmeelement **(42)** an der festen Fläche festzuhalten, und durch folgende Verfahrensschritte **gekennzeichnet** ist:
loses Einführen einer Manschette **(48)** in eine Bohrung **(43)** des Aufnahmeelements;
Einführen des vertikalen Rohrs in die Manschette, so dass ein Ende **(22)** des vertikalen Rohrs sich über eine ausgewählte Länge unterhalb des Aufnahmeelements erstreckt, wobei die Länge des vertikalen Rohrs, die sich unterhalb eines unteren Endes **(47)** des Aufnahmeelements erstreckt, variabel ist; und
Einziehen der Manschette in die Bohrung des Aufnahmeelements, so dass das vertikale Rohr zwischen dem Aufnahmeelement und der Manschette zusammenpressend eingeklemmt ist.

19. Verfahren nach Anspruch 18, weiterhin **gekennzeichnet durch**
den Schritt, der darin besteht, **durch** Anziehen einer auf einem Abschnitt **(55)** der Manschette vorgesehenen Mutter **(53)** die Manschette in die Bohrung einzuziehen.

20. Verfahren nach einem der Ansprüche 18 und 19, weiterhin **dadurch gekennzeichnet,**
**dass** die Manschette mehrere sich in Längsrichtung erstreckende, beabstandete Schlitze **(50)** umfasst, wobei beim Einziehen der Manschette in die Bohrung Abschnitte **(49)** der Manschette, die von den Schlitzen begrenzt werden, zusammengedrückt werden.

## Revendications

1. Système de suspension (10) permettant de suspendre un ou plusieurs éléments parmi des têtes d'éclairage (26), moniteurs (28, 30), lampes articulées (34), caméras, et autres appareils médicaux à partir d'une structure de plafond (12) à une hauteur sélectionnable, le système de suspension comprenant un tube descendant (20) configuré pour supporter un ou plusieurs éléments parmi des têtes d'éclairage, moniteurs, caméras, et autres appareils médicaux, **caractérisé par** :
un élément récepteur (42) qui reçoit le tube descendant de sorte qu'une extrémité distale (22) du tube descendant se prolonge au-dessous d'une extrémité inférieure (47) de l'élément récepteur ;
un collier (48) qui est reçu par l'élément récepteur et entoure une partie (41) du tube descendant pour effectuer un serrage compressif de la partie du tube descendant qui est située à l'intérieur de l'élément récepteur de sorte qu'une longueur du tube descendant qui se prolonge au-dessous de l'extrémité inférieure de l'élément récepteur est variable ; et
des moyens (14, 16) de montage de l'élément récepteur dans la structure de plafond.

2. Système de suspension selon la revendication 1, **caractérisé, en outre, par** :
les moyens de montage qui comprennent une plaque de montage (14) fixée de manière rigide à l'élément récepteur et configurée pour être montée sur la structure de plafond, la plaque de montage ayant une ouverture (45) à travers laquelle passe une extrémité du tube descendant.

3. Système de suspension selon la revendication 1 ou 2, **caractérisé, en outre, par** :
l'élément récepteur qui a un diamètre interne D qui diminue vers l'extrémité inférieure.

4. Système de suspension selon la revendication 3, **caractérisé, en outre, par** :
le diamètre interne de l'élément récepteur qui est défini par un alésage généralement tronconique (43).

5. Système de suspension selon l'une quelconque des revendications précédentes 1 à 4, **caractérisé, en outre, par** :
le collier qui a un diamètre externe d qui diminue vers une extrémité inférieure (52) de celui-ci.

6. Système de suspension selon la revendication 5, **caractérisé, en outre, par** :
le diamètre externe du collier qui est supérieur à un diamètre interne minimum de l'élément récepteur.

7. Système de suspension selon l'une quelconque des revendications précédentes 1 à 6, **caractérisé, en outre, par** :
un écrou de serrage (53) adapté à un filetage sur une partie (55) du collier de manière à faire avancer de manière compressive le collier dans l'élément récepteur.

8. Système de suspension selon l'une quelconque des revendications précédentes 1 à 7, **caractérisé, en outre, par** :
le collier qui comprend au moins une fente d'extension longitudinale (50).

9. Système de suspension selon la revendication 8, **caractérisé, en outre, par** :
le collier qui comprend une pluralité de fentes se prolongeant longitudinalement.

10. Système de suspension selon l'une quelconque des revendications précédentes 8 et 9, **caractérisé, en outre, par** :
la au moins une fente qui définit une bande flexible (49) qui dévie radialement vers l'intérieur lorsqu'une force de compression extérieure est appliquée au collier.

11. Système de suspension selon l'une quelconque des revendications précédentes 1 à 10, **caractérisé, en outre, par** :
le tube descendant qui comprend un cylindre ayant un diamètre extérieur généralement uniforme.

12. Système de suspension selon l'une quelconque des revendications précédentes 1 à 11, **caractérisé, en outre, par** :
une fusée de pivotement (32) disposée à une extrémité inférieure du tube descendant.

13. Système de suspension selon la revendication 12, **caractérisé, en outre, par** :
un bras articulé (24) fixé à la fusée de pivotement, le bras articulé comportant au moins une articulation réglable (36) et une extrémité de fixation (35) adaptée pour recevoir l'un des éléments parmi des têtes d'éclairage, moniteurs, lampes articulées, caméras, et autres appareils médicaux.

14. Système de suspension selon l'une quelconque des revendications précédentes 1 à 13, **caractérisé, en outre, par** :
une longueur de l'extrémité distale du tube descendant qui se prolonge au-dessous de l'élément récepteur qui est réglable sans palier vers les limites supérieures et inférieures.

15. Système de suspension selon l'une quelconque des revendications précédentes 1 à 14, **caractérisé, en outre, par** :
le collier qui comporte une surface extérieure essentiellement tronconique (48a), la surface extérieure tronconique étant en prise dans une surface intérieure essentiellement tronconique (43a) de l'élément récepteur.

16. Système de suspension selon l'une quelconque des revendications précédentes 1 à 14, **caractérisé, en outre, par** :
le collier qui comporte un alésage dont la forme permet de recevoir le tube descendant en le faisant coulisser à travers lui, en l'absence d'une force de compression appliquée au collier.

17. Système de suspension selon l'une quelconque des revendications précédentes 1 à 16, **caractérisé, en outre, par** :
le collier comprenant :
une surface extérieure dont le diamètre va en se rétrécissant se terminant à une extrémité étroite, que la surface extérieure cale de manière compressive au moins partiellement à l'intérieur d'un alésage axial de l'élément récepteur ; et
une surface intérieure qui est comprimée contre le tube descendant à un point choisi qui est sensible au calage afin de fixer le tube descendant dans l'alésage du tube descendant au point choisi.

18. Procédé permettant de régler de manière variable une distance par rapport à une surface fixe, d'une extrémité distale d'un tube descendant afin de supporter un dispositif médical, le procédé comprenant le fait de supporter de manière rigide un élément récepteur (42) à partir de la surface fixe, ce procédé étant **caractérisé par** les étapes consistant à :
insérer de manière lâche un collier (48) dans un alésage (43) de l'élément récepteur ;
insérer le tube descendant dans le collier de sorte qu'une extrémité (22) du tube descendant se prolonge sur une distance choisie au-dessous de l'élément récepteur, la distance du tube descendant qui se prolonge au-dessous d'une extrémité inférieure (47) de l'élément récepteur étant variable ; et
faire avancer le collier dans l'alésage de l'élément récepteur de sorte que le tube descendant est serré de manière compressive par l'élément récepteur et le collier.

19. Procédé selon la revendication 18, **caractérisé, en outre, en ce que** :
l'étape consistant à faire avancer le collier dans l'alésage comprend le serrage d'un écrou (53) sur une partie (55) du collier.

20. Procédé selon l'une ou l'autre des revendications 18 et 19, **caractérisé, en outre, en ce que**:
le collier comprend une pluralité de fentes (50) espacées qui se prolongent longitudinalement, l'étape consistant à faire avancer le collier dans l'alésage comprenant la mise en compression des parties (49) du collier définies entre les fentes.
